(19) Europäisches Patentamt
European Patent Office
Office européen des brevets

(11) Numéro de publication : 0 344 202 B1

(12) # FASCICULE DE BREVET EUROPEEN

(45) Date de publication du fascicule du brevet : **05.08.92 Bulletin 92/32**

(51) Int. Cl.$^5$ : **C08B 37/02,** A61K 49/04

(21) Numéro de dépôt : **88901856.0**

(22) Date de dépôt : **12.02.88**

(86) Numéro de dépôt international : **PCT/FR88/00077**

(87) Numéro de publication internationale : **WO 88/06162 25.08.88 Gazette 88/19**

(54) **POLYMERES IODES, LEURS PROCEDES DE PREPARATION ET LEURS APPLICATIONS COMME PRODUITS DE CONTRASTE.**

(30) Priorité : **13.02.87 FR 8701876**

(43) Date de publication de la demande : **06.12.89 Bulletin 89/49**

(45) Mention de la délivrance du brevet : **05.08.92 Bulletin 92/32**

(84) Etats contractants désignés : **AT BE CH DE FR GB IT LI LU NL SE**

(56) Documents cités :
**FR-A- 2 362 117**
**US-A- 2 811 516**
**CHEMICAL ABSTRACTS, vol. 75, 1971, Columbus, Ohio, US, page 238, abstract no. 67490b**

(73) Titulaire : **GUERBET S.A.**
**15, rue des Vanesses**
**F-9360 Villepinte (FR)**

(72) Inventeur : **PARIS, Dominique**
**10, rue des Aulnes**
**F-9360 Aulnay-sous-Bois (FR)**
Inventeur : **NIGRETTO, Jean-Maxime**
**349, Parc de Cassan**
**F-95290 L'Isle-Adam (FR)**
Inventeur : **BONNEMAIN, Bruno**
**9, rue de Reims**
**F-77290 Mitry-Mory (FR)**
Inventeur : **MEYER, Dominique**
**23, rue du Tage**
**F-75013 Paris (FR)**
Inventeur : **DOUCET, Didier**
**141, avenue du Dr. Roosenfeld**
**F-93230 Romainville (FR)**

(74) Mandataire : **Le Guen, Gérard et al**
**CABINET LAVOIX 2, place d'Estienne d'Orves**
**F-75441 Paris Cédex 09 (FR)**

EP 0 344 202 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des polymères iodés utilisables en radiographie comme produits de contraste et notamment comme produits de contraste injectables.

Les composés utilisés comme produits de contraste injectables doivent non seulement absorber les rayons X mais également posséder diverses propriétés et notamment

- une hydrosolubilité suffisante aux pH physiologiques,
- une faible toxicité,
- une osmolarité convenable,
- une stabilité chimique dans l'organisme et à la stérilisation.

Jusqu'à présent il était nécessaire d'injecter aux patients des quantités importantes de produits de contraste pour obtenir un diagnostic correct à partir de l'image radiographique. Aussi, les recherches se sont elles orientées vers des dérivés à osmolarité plus appropriée et à toxicité réduite. Deux nouvelles catégories de produits ont ainsi fait leur apparition : les produits ioniques à faible osmolarité et les produits non ioniques.

Plus récemment on a cherché à développer une autre approche consistant à freiner la diffusion des produits opacifiants dans l'espace extra-vasculaire.

A cet effet on a proposé différents polymères iodés. C'est ainsi que US-A-3 852 341 décrit des polymères iodés obtenus par copolymérisation d'un acide 3,5-diacylamino-2,4,6-triiodobenzénique avec des composés bifonctionnels tels qu'un diépoxyde. Ces polymères sont solubles dans l'eau. FR-A-2 200 018 décrit des copolymères voisins qui sont réticulés et insolubles dans l'eau.

US-A-4 406 878 décrit un polymère iodé qui est obtenu par réaction d'anhydride tétraiodophtalique sur l'alcool polyvinylique et réticulation.

La présente invention vise à fournir de nouveaux polymères iodés qui se distinguent par une faible osmolarité et une bonne tolérance.

La présente invention a pour objet des polymères iodés caractérisés en ce qu'ils comprennent un squelette constitué par un dextrane sur lequel sont greffés des groupes de formule

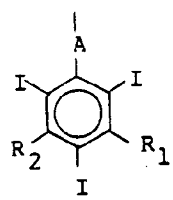

I

dans laquelle

A est un groupe formant un pont entre la chaine du dextrane et le noyau benzénique,

$R_1$ est un groupe choisi parmi le groupe COOH, le groupe COOH salifié par une base pharmaceutiquement acceptable et les groupes de formule et

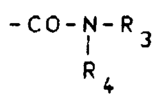

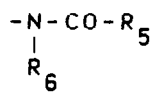

,

et

$R_2$ est un groupe choisi parmi les groupes de formule et

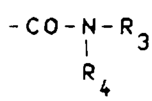

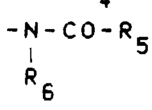

formules dans lesquelles

$R_3$ et $R_5$ sont choisis parmi les groupes alkyle en $C_1$ à $C_6$, hydroxyalkyle en $C_1$ à $C_6$, polyhydroxyalkyle en $C_1$ à $C_6$, alkoxy ($C_1$-$C_6$) alkyle ($C_1$-$C_6$) et alkoxy ($C_1$-$C_6$) hydroxyalkyle ($C_1$-$C_6$),

$R_4$ et $R_6$ sont choisis parmi un atome d'hydrogène et les groupes alkyle en $C_1$ à $C_6$, hydroxyalkyle en $C_1$ à $C_6$, polyhydroxyalkyle en $C_1$ à $C_6$, alkoxy ($C_1$-$C_6$) alkyle ($C_1$-$C_6$) et alkoxy ($C_1$-$C_6$) hydroxyalkyle ($C_1$-$C_6$), $R_3$ pouvant être en outre un groupe de formule

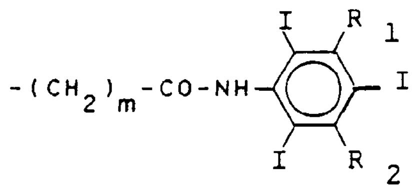

m étant un entier de 1 à 6 et

$R_1$ et $R_2$ ayant la signification donnée précédemment.

Le dextrane qui sert de support aux groupes triiodobenzéniques est un polymère obtenu généralement à partir de saccharose par action de Leuconostoc mesenteroïdes.

Les dextranes sont des polymères constitués par des unités d ' α -D-glucose. Les unités glycosyle des chaînes sont liées par des enchaînements essentiellement 1 → 6 selon le schéma suivant

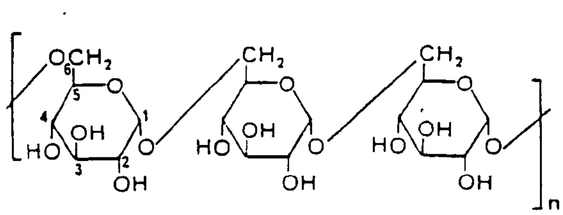

Les dextranes du commerce ont généralement subi une hydrolyse partielle suivie d'un fractionnement pour obtenir des polymères plus homogènes du point de vue de la masse moléculaire.

Les dextranes utilisés dans la présente invention peuvent avoir notamment des masses de 3.000 à 150.000 et ont de préférence des masses de 10.000 à 100.000 pour fournir des polymères iodés suffisamment hydrosolubles.

Les polymères selon l'invention peuvent être obtenus par greffage d'un motif triiodobenzénique sur la chaîne d'un dextrane.

Un tel greffage peut être réalisé :

– soit directement par réaction des groupes hydroxy du dextrane avec un réactif tel qu'un chlorure d'acide ou un anhydride d'acide à groupe triiodobenzénique.

– soit indirectement par conversion, des groupes hydroxy du dextrane en des groupes plus réactifs, puis réaction de ces groupes plus réactifs avec un dérivé triiodobenzènique approprié.

Dans le premier cas, on obtient des polymères à groupes esters selon le schéma suivant

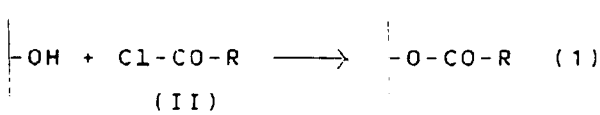

(II) (1)

La réaction est avantageusement effectuée en activant au préalable le dextrane par une base telle que le terbutylate de potassium, l'hydrure de sodium, ou le 1,4-diazabicyclo [2,2,2] octane (DABCO) ou des alcoolates de sodium tels que méthylate, éthylate de sodium.

Le chlorure d'acide iodé peut être notamment un chlorure d'acide 2,4,6-triiodobenzoique de formule

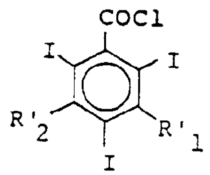

IIa

dans laquelle

R'$_1$ et R'$_2$ sont des groupes choisis parmi un groupe de formule

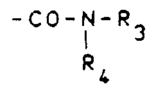

et un groupe de formule

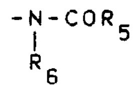

$R_3$, $R_4$, $R_5$ et $R_6$ ayant la signification donnée précedemment.

La réaction avec un chlorure d'acide de formule IIa conduit à un polymère sur lequel sont greffés des groupes

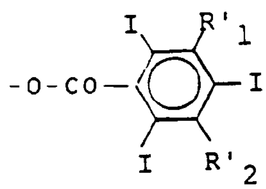

III

La réaction peut être effectuée dans un solvant polaire.

Dans les réactions de greffage indirect on effectue dans une première étape en quelque sorte une activation du polymère conduisant à la conversion des groupes hydroxy peu réactifs en des groupes plus réactifs, capables de réagir avec des molécules triiodobenzéniques ayant notamment des groupes $NH_2$.

Comme réactions d'activation on peut utiliser notamment les méthodes suivantes :

a) Méthode au périodate de sodium

Cette méthode consiste à faire réagir le périodate de sodium sur un dextrane de façon à obtenir un dextrane comprenant des motifs de structure

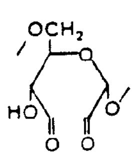

La réaction de ce dialdéhydodextrane avec une amine $RNH_2$, puis la réduction des groupes obtenus conduisent à un dextrane sur lequel sont greffés des groupes N-R, selon le schéma suivant

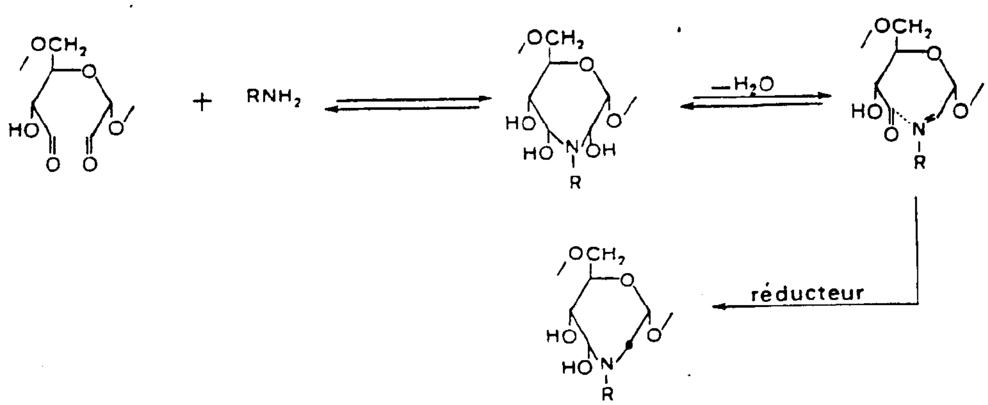


b) Méthode au tosylate ou au mésylate

Cette méthode consiste à faire réagir le chlorure de mésyle avec un dextrane pour fournir un mésylate selon le schéma suivant

$$\vdash OH + ClSO_2CH_3 \longrightarrow \vdash OSO_2CH_3 + HCl$$

La réaction du polymère obtenu avec une amine $RNH_2$ conduit à un dextrane sur lequel sont greffés des groupes -NHR, selon le schéma suivant

$$\vdash OSO_2Z + RNH_2 \longrightarrow \vdash NHR + HOSO_2Z$$

c) Méthode à l'acide monochloroacétique

Cette méthode consiste à faire réagir l'acide monochloroacétique en milieu alcalin selon le schéma réactionnel suivant

$$\vdash OH + ClCH_2COONa \xrightarrow{OH^-} \vdash OCH_2COON_a + Cl^- + H_2O$$

La réaction du carboxyméthyl dextrane obtenu avec une amine $RNH_2$ conduit à un dextrane sur lequel sont greffés des groupes $-OCH_2-CO-NHR$ selon le schéma suivant

$$\vdash OCH_2COONa + RNH_2 \longrightarrow \vdash OCH_2-CO-NH-R$$

En pratique la réaction nécessite un agent de couplage. A cet effet on peut utiliser des agents de couplage classiquement utilisés pour la synthèse des peptides tels que les carbodiimides et la N-éthoxy carbonyl-2-éthoxy-1,2-dihydroquinoléine (EEDQ). On peut également utiliser comme agent de couplage l'hexaméthyldisilazane de formule $(CH_3)_3SiNHSi(CH_3)_3$.

d) Méthode à l'épichlorhydrine (voie alcaline)

Cette méthode consiste à faire réagir l'épichlorhydrine en milieu alcalin selon le schéma suivant

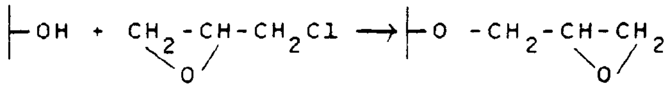

La réaction du polymère obtenu avec une amine $RNH_2$ conduit à un dextrane sur lequel sont greffés des groupes

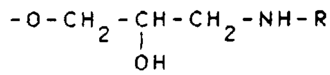

selon le schéma suivant

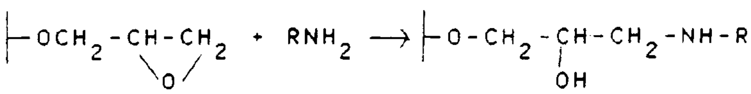

e) Méthode à l'épichlorhydrine (voir catalytique)

$e_1$) Cette méthode consiste à faire réagir l'épichlorhydrine avec le dextrane en présence de Zn $(BF_4)_2$ selon le schéma suivant

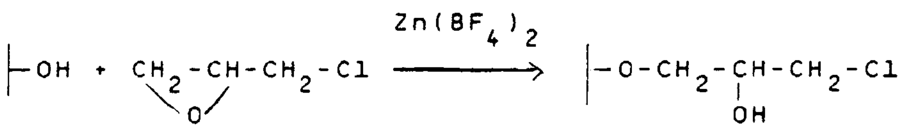


La réaction du polymère obtenu avec une amine a lieu selon le schéma suivant

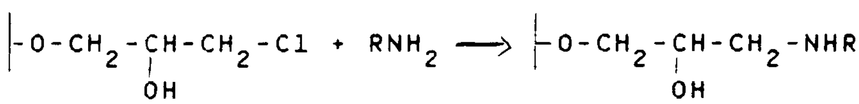

$e_2$) On peut de même faire réagir le polymère avec l'ammoniaque selon le schéma suivant

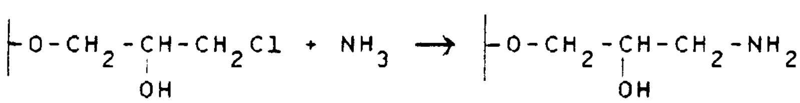

On peut alors faire réagir un chlorure d'acide RCOCl sur l'amino-3-hydroxy-2-propyl-dextrane ainsi obtenu, conduisant à une structure de type

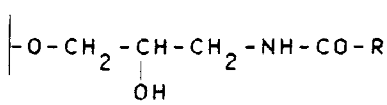

f) Méthode à l'anhydride succinique.

Cette méthode consiste à faire réagir l'anhydride succinique dans un solvant polaire sur le dextrane en utilisant la 4-diméthylaminopyridine comme catalyseur d'acylation, selon le schéma réactionnel suivant

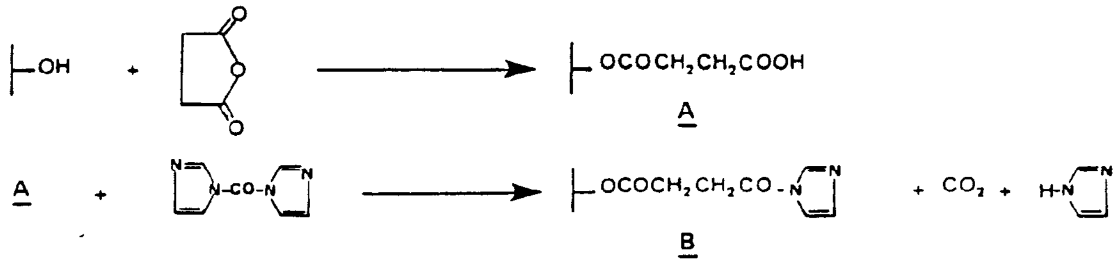

Le polymère B activé peut réagir ensuite avec une amine $RNH_2$ selon le schéma réactionnel suivant

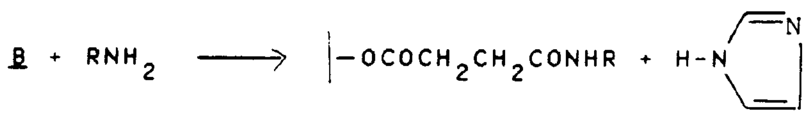

Les amines iodés utilisées dans ces réactions peuvent être notamment des amines de formule

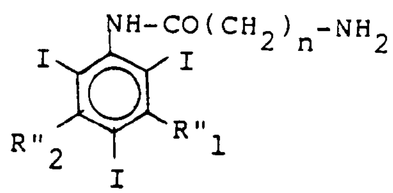

IVa

dans laquelle n est un entier de 1 à 5,

R"1 est un groupe -COOH, un groupe COOH salifié par une base pharmaceutiquement acceptable ou un groupe

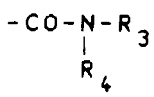

R"2 est un groupe

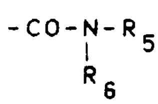

ou un groupe

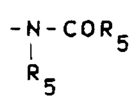

$R_3$, $R_4$, $R_5$ et $R_6$ ayant la signification donnée précédemment.

Des amines de ce type sont décrites notamment dans FR-A-227 640. Comme exemple de telles amines on peut citer les amines de formules suivantes

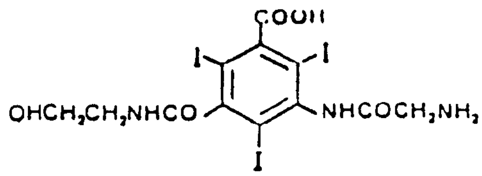

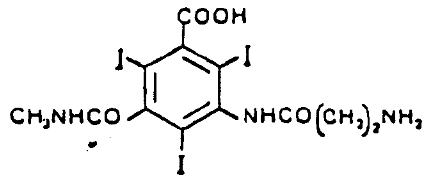

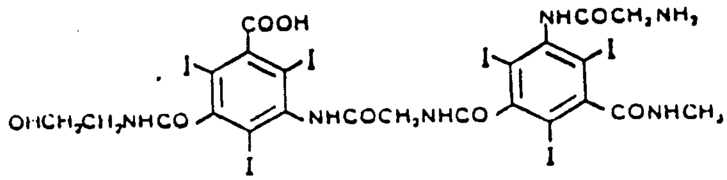

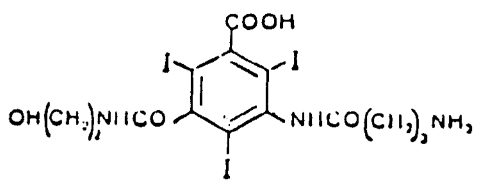

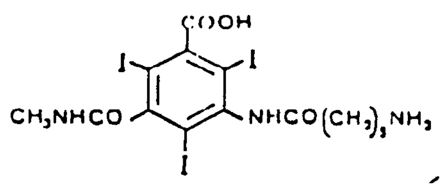

Ces amines conduisent à des dextranes possédant des groupes greffés de formule

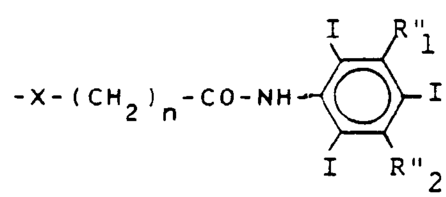

V

dans laquelle n, $R''_1$ et $R''_2$ ont la signification donnée précédemment et

X est un groupe tel que

$N-$ , $-NH-$ , $-O-CH_2-CO-NH-$ ,

$-O-CH_2-\underset{\displaystyle OH}{\underset{|}{CH}}-CH_2-NH-$ et

$-OCOCH_2CH_2-CO-NH-$

Les chlorures d'acide de formule IIa utilisés dans la méthode $e_2$ conduisent à des dextranes possédant des groupes greffés de formule

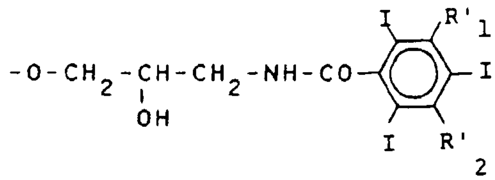

VI

dans laquelle R′ et $R'_2$ ont la signification donnée précédemment.

Les exemples suivants illustrent la préparation des polymères iodés.

Dans ces exemples les taux d'iode ont été mesurés par :

* analyse élémentaire
* argentimètrie
* spectroscopie UV

De plus, la plupart des produits ont été caractérisés par dosage acide-base dans différents solvants (eau/eau-acétone/DMSO).

Toutes les masses moléculaires ont été mesurées par chromatographie d'exclusion stérique sous haute pression, désignée également chromatographie par perméation de gel (GPC) en comparaison avec les dextranes de départ. Les masses ainsi déterminées sont désignées $M_{GPC}$ dans ce qui suit.

Exemple 1

20 g de dextrane (Dextran T40, de Pharmacia Fine Chemicals, masse moléculaire moyenne en poids indiquée Mw = 40 000, $M_{GPC}$ = 27 000) sont dissous dans 165 ml d'une solution aqueuse de soude 6M à 0°C. La solution est agitée à cette température pendant 20 minutes.

41 g d'acide monochloroacétique sont ajoutés dans le milieu réactionnel. La température est alors portée à 60°C et la solution est maintenue 20 minutes à cette température sous agitation. Le mélange est ensuite refroidi puis neutralisé à pH 7,00 par addition de HCl concentré. Le produit est alors précipité dans 1 litre de méthanol, filtré, lavé et séché à 50°C sous vide.

24 g de carboxyméthyldextrane, sel de sodium sont obtenus, avec un degré de substitution de 50% (déterminé par dosage acide-base).

Cette même opération est répétée une seconde fois; 26 g de carboxyméthyldextrane, sel de sodium sont obtenus avec un degré de substitution de 80%.

La masse moléculaire du polymère obtenu, déterminé par chromatographie d'exclusion stérique est de 47 000.

2 g de carboxyméthyldextrane à 3,50 meq/g de fonctions -COONa (80% de substitution) sont dissous dans 10 ml d'eau et le pH du milieu est porté à 2,50 par addition de HCl concentré. Par ailleurs, 2,6 g de N-éthoxycarbonyl-2 éthoxy-1,2 dihydroquinoléine (EEDQ) sont dissous dans 21 ml déthanol et ajoutés progressivement au milieu réactionnel, sous agitation de manière homogène. Après 30 minutes, 4,6 g d'amine iodée de formule

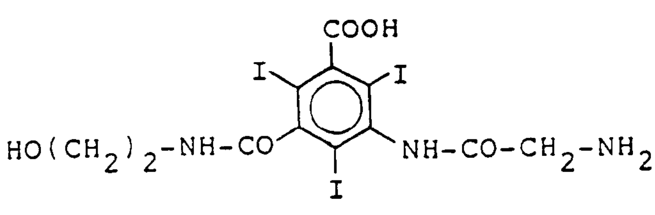

(VII)

(acide 2,4,6 triiodo-3-N-hydroxyéthylcarbamoyl-5-acétamido benzoique) dissoute dans 3,5 ml de soude 2M sont ajoutés au milieu réactionnel, le pH étant fixé à 8,50.

La réaction est laissée sous agitation à température ambiante pendant 4 heures. Puis le milieu réactionnel est évaporé sous vide pour éliminer l'éthanol avant d'être précipité dans 200 ml de méthanol. Le produit est séché sous vide à 50°C.

Le taux d'iode sur le polymère a été estimé à 6,2%.

Une seconde fixation est conduite avec les mêmes quantités de réactif pour aboutir à un taux d'iode de 16%.

Une troisième fixation avec 1 eq de EEDQ et 0,9 eq d'amine par rapport au carboxyméthyldextrane de départ permet d'amener le taux d'iode à 24%. Après avoir été séché sous vide, le polymère est redissous dans

l'eau, ultrafiltré et lyophilisé.

1,5 g de carboxyméthyldextrane iodé sous forme de sel de sodium sont obtenus avec une masse moléculaire de 48000 (déterminé par GPC). Le polymère obtenu présente des groupes greffés de formule

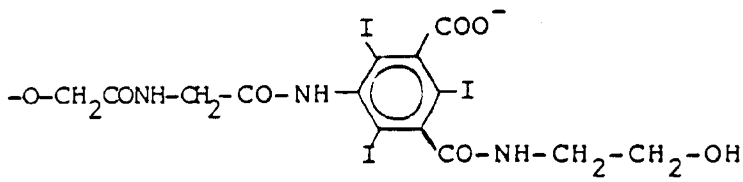

Exemple 2

1,5 g de carboxyméthyldextrane (précédemment obtenu dans l'exemple 1) à 3,50 meq/g de fonctions -COOH sous la forme acide sont dissous dans un mélange DMAC (20ml)/$H_2O$ (10 ml); 3,4 g de dicyclohexycarbodiimide dissous dans 10 ml de DMAC sont ajoutés à la solution précédente.

Quand le mélange est homogène, 3,6 g d'amine iodée de formule VII et 0,55 ml de triéthylamine dissous dans 10 ml d'eau sont ajoutés progressivement. La solution est laisée sous agitation à température ambiante pendant 24 heures, puis filtrée pour éliminer l'amine et la dicyclohéxylurée précipitées.

Le filtrat est évaporé à sec sous vide, repris à l'eau puis ultrafiltré et lyophilisé, (le sel de triéthylamine étant déplacé par de la soude).

On obtient 1,2 g de polymère, contenant 8% d'iode, avec une masse moléculaire de 48000 (déterminée par GPC).

Exemple 3

2 g de carboxyméthyldextrane (obtenu dans l'exemple 1) à 3,50 meq/g de fonctions -COONa dissous dans 5 ml d'eau et 1,5 g d'amine iodée de formule VII dissous dans 20 ml de soude 1N sont mélangés. Le pH du milieu est porté à 3,00 par addition de HCl.

Après 10 minutes, 1,6 g de chlorure de 1-éthyl-3(3-diméthylaminopropyl)-carbodiimide sont ajoutés. Le pH est maintenu constant entre 4,5 et 5,0 pendant la première heure de réaction.

La solution est laissée sous agitation à température ambiante pendant 24 heures, puis elle est filtrée. Le filtrat est ultrafiltré et lyophilisé.

On obtient 1,9 g de polymère contenant 15% d'iode, avec une masse moléculaire de 54 000 (déterminée par GPC).

Exemple 4

Le mode opératoire est identique à l'exemple 3 mais l'amine utilisée est la suivante

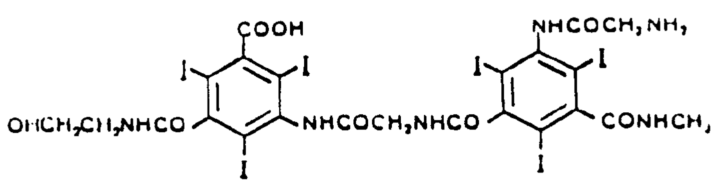

(2,9 g sont mis en réaction).

On obtient 2,3 g de polymère iodé contenant 24,6 % d'iode, avec une masse moléculaire de 62 000 (déterminée par GPC).

Exemple 5

On prépare un carboxyméthyldextrane comme à l'exemple 1 mais en utilisant comme polymère de départ du Dextran T18 (Pharmacia Fine Chemicals, Mw = 18 000, $M_{GPC}$ = 14 000).

Le carboxyméthyldextrane est obtenu avec un degré de substitution de 70 % et avec une masse moléculaire de 29 000 (déterminée par GPC).

2 g de carboxyméthyldextrane à 3,20 meq/g de fonctions -COONa sont dissous dans 10 ml d'eau et le pH

du milieu est porté à 2,50 par addition de HCl concentré. Par ailleurs, 2,4 g de N-éthoxycarbonyl-2 éthoxy-1,2-dihydroquinoléine sont dissous dans 20 ml d'éthanol et ajoutés progressivement au milieu réactionnel, sous agitation de manière homogène.

Après 30 minutes, 5,9 g d'amine iodée de formule VII dissous dans 4,5 ml de soude 2M sont ajoutés au milieu réactionnel, le pH étant fixé à 8,50. Le mode opératoire est ensuite identique à l'exemple 1.

Le taux d'iode sur le polymère a été estimé à 6,2 %.

Une seconde fixation (1,4 eq de EEDQ et 1,1 eq d'amine par rapport au carboxyméthyldextrane) per-met d'obtenir un taux d'iode de 16%, une troisième fixation (1,3 eq de EEDQ et 1,0 eq d'amine par rapport au CMD) conduit à un taux d'iode de 24% sur le polymère.

Après avoir été séché sous vide, le polymère est redissous dans l'eau, ultrafiltré et lyophilisé.

1,9 g de carboxyméthyldextrane iodé sous forme de sel de sodium sont obtenus, avec une masse moléculaire de 32 000 (déterminée par GPC).

Exemple 6

On prépare un carboxyméthyldextrane comme à l'exemple 1 mais on utilise du Dextran T10 (Pharmacia Fine Chemicals, Mw = 10 000, $M_{GPC}$ = 7 400).

Le carboxyméthyldextrane est obtenu avec un degré de substitution de 75% (3,5 meq/g) et avec une masse moléculaire de 15 000 (déterminée par GPC) alors que celle du dextrane de départ est de 7 400.

2 g de carboxyméthyldextrane à 3,35 meq/g de fonctions -COONa sont dissous dans 10 ml d'eau et le pH du milieu est porté à 2,50 par addition de HCl concentré. Par ailleurs, 2,5 g de N-éthoxycarbonyl-2 éthoxy-1,2-dihydroquinoléine sont dissous dans 20 ml d'éthanol et ajoutés progressivement au milieu réactionnel, sous agitation de manière homogène.

Après 30 minutes, 4,6 g d'amine iodée de formule VII dissous dans 3,5 ml de soude 2M sont ajoutés au milieu réactionnel, le pH étant fixé à 8,50.

Le mode opératoire est ensuite identique à l'exemple 1.

Le taux d'iode sur le polymère est estimé à 13%.

Une seconde fixation (1,4 eq de EEDQ et 0,9 eq d'amine par rapport au carboxyméthyldextrane) permet d'obtenir un taux d'iode de 22%; une troisième fixation (1,0 eq de EEDQ et 1,0 eq d'amine par rapport au carboxyméthyldextrane) conduit à un taux d'iode de 26% sur le polymère.

Après avoir été séché sous vide, le polymère est redissous dans l'eau, ultrafiltré et lyophilisé.

1,8 g de carboxyméthyldextrane iodé sous forme de sel de sodium sont obtenus, avec une masse moléculaire de 15 000 (déterminée par GPC).

Exemple 7

0,25 g de carboxyméthyldextrane (obtenu dans l'exemple 1) à 3,50 meq/g de fonctions -COOH mises sous la forme acide sont dissous dans 10 ml de diméthylformamide, puis sont ajoutés 3 ml d'hexaméthyldisilazane. Le milieu réactionnel est porté à 100°C pendant 16 heures, puis celui-ci est évaporé et repris deux fois dans le DMF.

1,4 g d'amine iodée de formule VII sont alors ajoutés, le chauffage est porté à 50°C pendant 6 heures. Après refroidissement, le milieu réactionnel est dilué dans 100 ml d'eau. Après 1 heure, celui-ci est concentré puis précipité dans le méthanol.

Après avoir été séché sous vide, le polymère est redissous dans l'eau, ultrafiltré et lyophilisé.

On obtient 0,2 g de carboxyméthyldextrane iodé sous forme de sel de sodium avec un taux d'iode de 19,8% et une masse moléculaire de 48 000 (déterminée par GPC).

Exemple 8

2 g de carboxyméthyldextrane à 3,20 meq/g de fonctions -COONa (obtenu à partir de dextrane de Mw 40 000) sont dessous dans 10 ml d'eau, le pH du milieu est porté à 2,50 par addition de HCl concentré. Par ailleurs, 2 équivalents de N-éthoxycarbonyl-2 éthoxy 1-2-dihydroquinoléine et 1,5 eq d'amine iodée de formule VII sont ajoutés selon le mode opératoire décrit dans l'exemple 1.

Une deuxième fixation est conduite avec 1,5 eq d'EEDQ et 1,5 eq d'amine par rapport au carboxyméthyldextrane de départ.

On obtient 1,5 g de polymère iodé contenant 35% d'iode, avec une masse moléculaire de 50 000.

Exemple 9

3 g de dextrane (Mw = 40 000, MGPC = 27 000) sont dissous dans 30 ml de DMSO, 7,2 g d'anhydride succinique dans 35 ml de DMSO sont ajoutés à la solution précédente ainsi que 1,8 g de 4-diméthylaminopyridine sous forme solide. La solution est agitée pendant 4 heures à 45°C. Le milieu réactionnel est précipité dans du méthanol. Après séchage, il est redissous dans de l'eau puis ultrafiltré et lyophilisé. 3 g de polymère sont obtenus avec un taux de substitution de 131%.

1 g du polymère précédemment obtenu à 4,87 meq/g de fonctions acides est dissous dans 20 ml de DMSO avec 1,6 g de carbonyldiimidazole.

La solution est agitée à température ambiante pendant 30 minutes. Puis 6 g d'amine iodée de formule VII dissous dans 4,5 ml de soude 2M sont ajoutés; l'agitation est laissée pendant 24 heures. Le milieu réactionnel est précipité dans du méthanol. Après séchage, le produit est redissous dans d l'eau, ultrafiltré et lyophilisé.

Le polymère obtenu possède un taux d'iode de 20%.

Le polymère obtenu présente des groupes greffés de formule

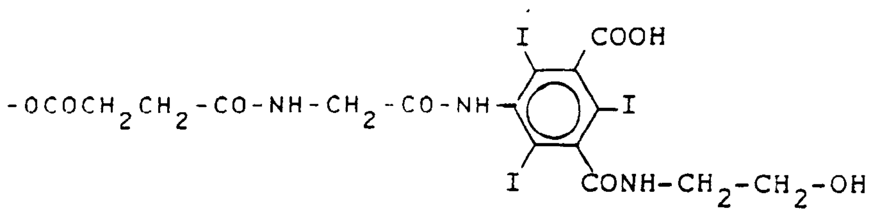

Exemple 10

2 g de Dextran T 40 (Pharmacia Fine Chemicals, Mw = 40 000, MGPC= 27 000) sont dissous dans 10 ml de DMSO anhydre par un léger chauffage à 40°C.

2,8 g de tertbutylate de potassium dissous dans 10 ml de DMSO sont ajoutés et la solution est maintenue sous agitation à 40°C pendant une heure.

8 g de chlorure de l'acide 2,4,6-triiodo-3-N-méthylacétamido-5-N-méthylcarbamoyl benzoique dissous dans 20 ml de DMSO sont alors introduits et la température est portée à 60°C pendant 5 heures.

Le polymère est alors précipité dans 150 ml de méthanol; après séchage, il est redissous dans de l'eau, ultrafiltré et lyophilisé.

On obtient 1,65 g de polymère ayant un taux diode de 13,6 %; et une masse moléculaire de 16 500 (déterminée par GPC).

Le polymère obtenu présente des groupes greffés de formule

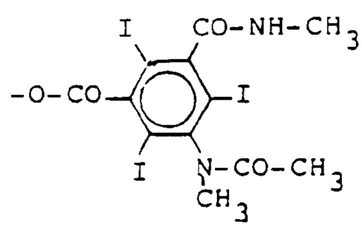

Exemple 11

2 g de dextrane (Mw = 40 000, $M_{GPC}$ = 27 000) sont dissous dans 10 ml de DMSO anhydre à 40°C; 1,4 g de tertbutylate de potassium dissous dans 5 ml de DMSO sont ajoutés et la solution est maintenue sous agitation à 40°C pendant une heure. 9,6 g du chlorure d'acide utilisé à l'exemple 10 dissous dans 25 ml de DMSO sont alors introduits et la température est portée à 60°C pendant 5 heures. Le traitement est ensuite identique à l'exemple 10.

Le taux d'iode présent sur le polymère est égal à 10,0%; la masse moléculaire du polymère est de 16 000.

Une seconde fixation est conduite sur le dérivé obtenu avec 1 eq de base et 1 eq de chlorure d'acide par rapport au dextrane de départ. On obtient 1,5 g de polymère contenant 16,5 % d'iode avec une masse moléculaire de 14 000.

Exemple 12

2,5 g de dextrane (Dextran T80, Pharmacia Fine Chemicals, Mw = 80 700, $M_{GPC}$ = 48 000) sont dissous dans 15 ml de DMSO anhydre à 40°C;

0,75 g d'hydrure de sodium dispersé à 50% dans de l'huile sont ajoutés et la solution est maintenue sous agitation à 40°C pendant 90 minutes. 11,7 g de chlorure d'acide utilisé à l'exemple 10 dissous dans 20 ml de DMSO sont alors introduits et la température est portée à 60°C pendant 5 heures.

Le polymère est précipité dans 150 ml de méthanol; après séchage, il est redissous dans de l'eau, ultrafiltré et lyophilisé.

On obtient 2,2 g de polymère contenant 11,7 % d'iode avec une masse moléculaire de 28.000 (déterminé par GPC).

Exemple 13

2,5 g de dextrane (Dextran T18, Pharmacia Fine Chemicals, Mw = 18 000, $M_{GPC}$ = 14 000) sont dissous dans 20 ml de DMSO anhydre, à 40°C.

0,75 g d'hydrure de sodium dispersé à 50% dans de l'huile sont ajoutés et la solution est maintenue sous agitation à 40°C pendant 1 heure. 11,7 g de chlorure d'acide utilisé à l'exemple 10 dissous dans 25 ml de DMSO sont introduits et la température est portée à 60°C pendant 6 heures.

Après traitement, le taux diode présent sur le polymère est égal à 10,0%, la masse moléculaire du polymère est de 13 000.

Une seconde fixation est conduite sur le dérivé obtenu avec 1 eq de base.

On obtient 2 g de polymère contenant 13% d'iode, avec une masse moléculaire de 11 000.

Exemple 14

4 g de dextrane (Dextran T 40, Pharmacia fine Chemicals, Mw = 40 000, $M_{GPC}$ = 27 000) sont mis en solution dans 60 ml de pyridine et la température du milieu est portée à 70°C. 5,66 g de chlorure de mésyle sont ajoutés lentement au milieu réactionnel, puis environ 50 ml de pyridine, la température est portée à 100°C.

Après 2 heures 30 de réaction, la solution est précipitée dans 400 ml de méthanol. Le produit est filtré puis séché sous vide à 50°C.

On obtient 3,7 g de polymère avec un taux de substitution de 18% en motifs mésylate (%S = 3,25).

1 g du mésylate précédemment obtenu à 1,02 meq/g de groupes mésyle est dissous dans 20 ml d'eau.

5 g d'amine de formule amine iodée ((2,4,6-triiodo-3-carboxy-5-N-hydroxyéthylcarbamoylphényl)-carbamoylméthylamine) dissous dans 3,8 ml de soude 2M sont ajoutés à pH = 9,60. La température est portée à 65°C pendant 6 heures puis le milieu réactionnel est laissé 12 heures à température ambiante.

La solution est précipitée dans 100 ml de méthanol; après filtration et séchage sous vide, le produit est redissous dans de l'eau puis ultrafiltré et lyophilisé.

On obtient 0,6 g de polymère contenant 2% d'iode avec une masse moléculaire de 24 000. Le polymère obtenu présente des groups greffés de formule

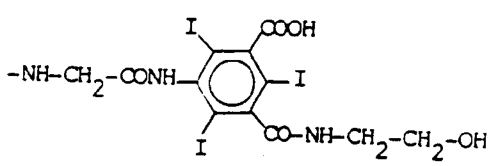

Exemple 15

1 g de dextrane (Dextran T10, Mw = 10 000, $M_{GPC}$ = 7 400) est dissous dans 20 ml d'une solution d'acétate de sodium 0,1 M avec 62 ml d'une solution de periodate de sodium 0,05 M, sous agitation. Après 40 minutes de réaction, 0,51 g de nitrate de plomb est ajouté; la solution est alors filtrée sur büchner.

2,1 g d'amine iodée ((2,4,6-triiodo-3-carboxy-5-N-hydroxyéthylcarbamoylphényl)carbamoylméthylamine) sont préalablement dissous dans une solution de soude 2N. Celle-ci est alors ajoutée progressivement au filtrat, le pH étant maintenu aux environs de 7,70. La solution est agitée pendant 16 heures à température ambiante. 0,25 g de borohydrure de sodium dissous dans 2 ml de soude 0,1N est alors ajouté; après deux heures, le pH est ramené au voisinage de la neutralité par addition d'acide chlorhydrique puis la solution est

ultrafiltrée et lyophilisée.

On obtient 1,9g de polymère avec un taux diode de 24,2% et une masse moléculaire $M_{GPC}$ de 7 000.

Le polymère obtenu présente des groupes greffés de structure

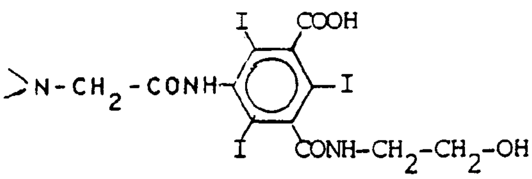

Exemple 16

Le mode opératoire est identique à l'exemple 15 mais le dextrane de départ est du Dextran T18 (Mw = 18 000, $M_{GPC}$ = 14 000). Le taux d'iode sur le polymère est de 23 %, avec une masse moléculaire $M_{GPC}$ de 17 000.

Exemple 17

Le mode opératoire est identique à l'exemple 15 mais le dextrane de départ est du Dextran T40 (Mw = 40 000, $M_{GPC}$ = 27 000). Le taux d'iode sur le polymère est de 24,9%, avec une masse moléculaire de 26 000.

Exemple 18

1,5 g de dextrane (Dextran T18, Mw = 18 000, $M_{GPC}$ = 14 000) sont dissous dans 30 ml d'une solution d'acétate de sodium 0,1 M avec 133 ml d'une solution de periodate de sodium 0,05 M. Après une heure de réaction, 1,15 g de nitrate de plomb sont ajoutés,; la solution est alors filtrée sur büchner.

4,75 g de la même amine qu'à l'exemple 15 sont préalablement dissous dans une solution de soude 2N. Celle-ci est alors ajoutée progressivement au filtrat, le pH étant maintenu aux environs de 8,00. La solution est agitée pendant 18 heures à température ambiante. 0,4 g de borohydrure de sodium dissous dans de la soude est agitée; après deux heures, le pH est ramené à la neutralité puis la solution est ultrafiltrée et lyophilisée.

On obtient 1,1 g de polymère avec un taux d'iode de 29,4% et une masse moléculaire $M_{GPC}$ de 17 000.

Exemple 19

2,5 g de dextrane (Mw = 40 000, $M_{GPC}$ = 27 000) sont dissous dans 5 ml d'une solution à 25% de tétrafluoroborate de zinc et 3,5 ml d'eau. 17 ml d'épichlorhydrine sont ajoutés au milieu réactionnel. Le mélange est agité à 80°C pendant 4 heures. La solution est ensuite précipitée dans 200 ml de méthanol. Le polymère obtenu est séché sous vide.

On obtient 2,4 g de polymère avec un taux de substitution de 12,6% en sites chlorés (% Cl = 2,57) et une masse moléculaire de 25 000.

Le polymère précédemment obtenu est dissous dans 7 ml d'ammoniaque et 7 ml d'eau. Le milieu réactionnel est agité à 40°C pendant 24 heures. La solution est alors précipitée dans 200 ml de méthanol, puis filtrée; le polymère obtenu est ensuite séché sous vide.

On obtient 2,3 g de polymère avec un taux de substitution de 12,8% en sites aminés (%N = 1,03) et une masse moléculaire de 20.000.

2,2 g de polymère à 0,74 meq/g de fonctions amines et 0,17 ml de triéthylamine sont dissous dans un mélange DMF/$H_2O$ (10 ml/9ml).

5,7 g de chlorure de l'acide 2,4,6-triiodo-3-N-méthylacétamido-5-N-méthylcarbamoylbenzoique dissous dans 33 ml de DMF sont ajoutés progressivement au milieu réactionnel et la température est portée à 60°C pendant 4 heures.

La solution est ensuite précipitée dans 200 ml de méthanol puis filtrée. Après avoir été séché sous vide, le polymère obtenu est redissous dans de l'eau, ultrafiltré puis lyophilisé.

On obtient 2,0 g de polymère contenant 8,1% d'iode, avec une masse moléculaire $M_{GPC}$ = 20 000. Le polymère obtenu présente des groupes greffés de formule

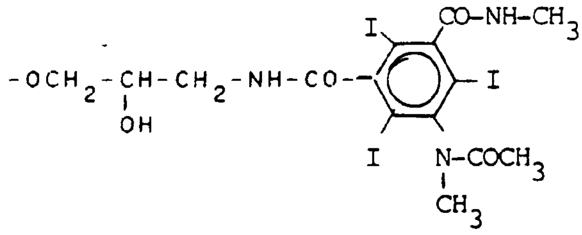

Exemple 20

Le mode opératoire est identique à l'exemple 19 mais le dextrane de départ est du Dextran T80 (Mw = 80 700, $M_{GPC}$ = 48 000). Le chloro-3 hydroxy-2-propyldextrane obtenu a un taux de substitution de 14,1% en sites chlorés (%Cl = 2,86) et une masse moléculaire de 42 000.

L'amino-3 hydroxy-2 propyldextrane obtenu a un taux de substitution de 11,6% en sites aminés (%N = 0,93) et une masse moléculaire de 30 000.

On obtient 2,0 g de polymère iodé contenant 5,8% d'iode et une masse moléculaire $M_{GPC}$ = 30 000.

Exemple 21

Le mode opératoire est identique à l'exemple 19 mais le dextrane de départ est du Dextran T18 (Mw = 18 000, $M_{GPC}$ = 14 000). Le chloro-3 hydroxy-2 propyl-dextrane obtenu a un taux de substitution de 16,4% en sites chlorés (%Cl = 3,29) et une masse moléculaire de 16 000. L'amino-3 hydroxy-2 propyldextrane a un taux de substitution de 15,0 % en sites aminés (%N = 1,20) et une masse moléculaire de 15 000.

On obtient 2,0 g de polymère iodé contenant 9,9 % d'iode et une masse moléculaire $M_{GPC}$ = 15 000.

Exemple 22

3,3 g de dextrane (Mw = 40 000, $M_{GPC}$ = 27 000) sont dissous dans 5 ml d'une solution à 25% de tétrafluoroborate de zinc et 3,3 ml d'eau. 17 ml dépichlorhydrine sont ajoutés au milieu réactionnel. Le mélange est agité à 80°C pendant 3 heures. La solution est ensuite précipitée dans 300 ml de méthanol. Le polymère obtenu est séché sous vide.

On obtient 3,1 g de polymère avec un taux de substitution de 7% en sites chlorés (%Cl = 1,44) et une masse moléculaire de 24 000.

0,5 g du polymère précédemment obtenu est dissous dans un mélange DMAC/$H_2O$ (7 ml/2 ml). 2,4 g d'amine iodée de formule VII, et 0,6 ml de triéthylamine dissous dans 6 ml DMAC/3 ml d'eau sont ajoutés au milieu réactionnel; la température est portée à 70°C pendant 4 heures.

La solution est alors précipitée dans 150 ml de méthanol puis filtrée; après séchage, le polymère est redissous dans de l'eau, ultrafiltré et lyophilisé.

On obtient 0,30 de polymère contenant 8,8% d'iode, avec une masse moléculaire $M_{GPC}$ = 30 000. Le polymère obtenu présente des groupes greffés de formule

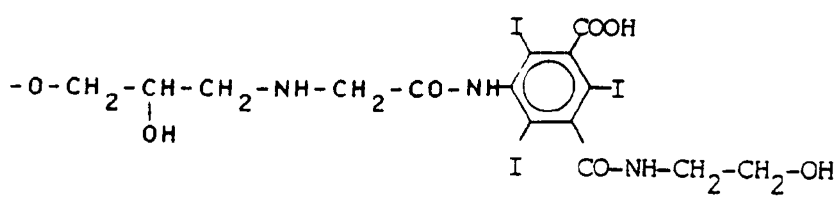

Exemple 23

0,3 g de chloro-3 hydroxy-2 propyldextrane (%cl = 1,44) obtenu comme à l'exemple 22 est dissous dans 20 ml de DMAC. 2,8 g d'amine de formule

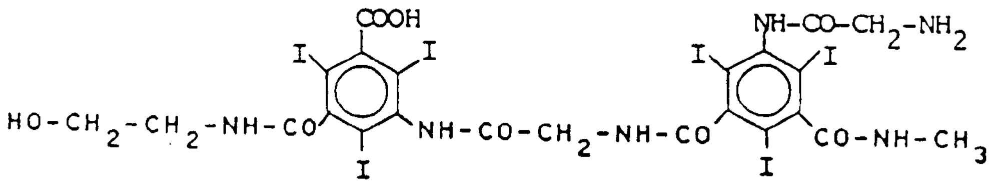

et 0,4 ml de triéthylamine dissous dans 10 ml d'eau sont ajoutés au milieu réactionnel, la température est portée à 60°C pendant 4 heures. La solution est alors précipitée dans 150 ml de méthanol puis filtrée après séchage, le polymère est redissous dans de l'eau, ultrafiltré et lyophilisé.

On obtient 0,2 g de polymère contenant 8,4% d'acide, avec une masse moléculaire $M_{GPC}$ = 28 000.

Exemple 24

5,0 g de dextrane (Mw = 40000, $M_{GPC}$ = 27000) sont dissous dans 8 ml de tampon 0,5 M en $CH_3COOHa$ et 0,5 M en $CH_3COOH$, le milieu réactionnel est porté à 70°C. A des intervalles de temps réguliers (t = 0, t = 2 heures, t = 4 heures), sont ajoutés 0,55 ml d'une solution à 50% de tétrafluoroborate de zinc et 9 ml dépichlorhydrine. Après la dernière addition, le chauffage est poursuivi pendant 5 heures. La solution est ensuite précipitée dans 500 ml de méthanol. Le polymère obtenu est séché sous vide.

On obtient 4,7 g de polymère avec un taux de substitution de 27,0 % en sites chlorés (% Cl = 5,2) et me masse moléculaire de 25000 ($M_{GPC}$).

Le polymère précédemment obtenu est dissous dans 20 ml d'une solution $NH_4OH2M,NH_4Cl2M$ ; la température est portée à 40°C. Le pH est ajusté à 9,50 par ajout d'ammonique.

Après 48 heures de chauffage, la solution est précipitée dans 400 ml de méthanol puis filtrée, le polymère obtenue est ensuite séché sous vide.

On obtient 4,0 g de polymère avec un taux de substitution de 28,0% en sites aminés (% N = 2,17) et une masse moléculaire de 20000.

4,0 g d'amino-3 hydroxy-2 propyldextrane sont dissous dans 6 ml d'eau avec 1,8 ml de triéthylamine ; le milieu réactionnel est porté à 60°C.

16,0 g de chlorure de l'acide 2,4,6-triido-3-N-méthylacétamido-5-N-méthylcarbamoylbenzoïque dissous 20 ml de DMAC sont ajoutés progressivement au milieu réactionnel et la température est laissée à 60°C pendant 24 heures. La solution est ensuite précipitée dans 400 ml de méthanol puis filtrée.

On obtient 3,0 g de polymère contenant 19,7% d'iode avec une masse moléculaire $M_{GPC}$ = 20000. Le polymère obtenu présente des groupes greffées de formule :

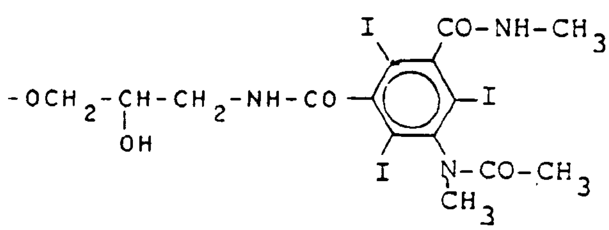

solubilité : 36,8 g/100ml soit 6,74 g $I_2$/100 ml
osmolalité à 37°C (tonométrie) : 288 mOsm/kg.

Exemple 25

5,0 g de dextrane (Mw = 40000, $M_{GPC}$ = 27000) sont dissous dans 8 ml de tampon 0,5 M en $CH_3COONa$ et 0,5 M en $CH_3COOH$ ; le milieu réactionnel est porté à 70°C. A des intervalles de temps réguliers (t = 0, t = 2 heures, t = 4 heures), sont ajoutés 0,55 ml d'une solution à 50% de tétrafluoroborate de zinc et 9 ml dépichlorhydrine. Après la dernière addition, le chauffage est poursuivi pendant 5 heures. La solution est ensuite précipitée dans 500 ml de méthanol. Le polymère obtenu est séché sous vide.

On obtient 4,7 g de polymère avec un taux de substitution de 32,0 % en sites chlorés (% Cl = 6,0) et une masse moléculaire $M_{GPC}$ de 25000.

2 g du polymère précédemment obtenu est dissous dans 5 ml d'eau à température ambiante.

8 g d'amine iodée de formule VII dissoute dans la quantité stoéchiométrique de soude est additionnée goutte à goutte au milieu réactionnel, en vérifiant que le pH soit toujours supérieur à 9,5.

La température est portée à 60°C pendant 24 heures.

La solution est précipitée dans 300 ml de méthanol puis filtrée ; après séchage, le polymère est redissous dans de l'eau, ultrafiltré et précipité dans un mélange méthanol-isopropanol.

On obtient 1,5 g de polymère contenant 22,6% d'iode, avec une masse moléculaire $M_{GPC}$ = 30000. Le polymère obtenu présente des groupes greffés de formule

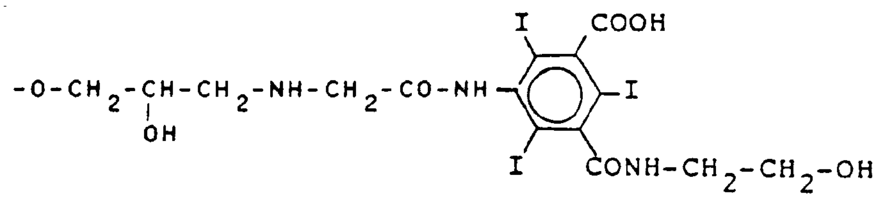

solubilité : 32,5 g/100 ml soit 7,9 g $I_2$/100 ml
osmolalité à 20°C : 474 mOsm/kg.

On donnera ci-après des résultats relatifs à l'hydrosolubilité des polymères ainsi préparés :

Tableau I

Solubilité dans l'eau

| Exemple | Solubilité % m/v | % iode en solution |
|---|---|---|
| 5 | 32,6 | 7,8 |
| 6 | 33,7 | 8,7 |
| 8 | 18,3 | 6,4 |
| 10 | 21,8 | 2,9 |
| 19 | 31,6 | 2,5 |
| 20 | 8,10 | 0,47 |
| 21 | 26,3 | 2,6 |

On donnera ci-après des résultats de l'étude pharmacologique

1) Etude pharmacocinétique

On a injecté à des lapins le polymère A obtenu à l'exemple 5 sous forme de solution à 8,64 gl/100 ml pendant 120 s et de l'Hexabrix (solution de sels de sodium et de méthylglucamine de l'acide ioxaglique) dilué à 8,64 gl/100 ml pendant 90s.

Les quantités injectées correspondent à 150 mgI/kg.

Les taux plasmatiques sont reportés sur le graphique I. Ils mettent en évidence la persistance d'un taux d'iode nettement plus important pour le polymère que pour l'Hexabrix.

2) Excrétion urinaire et biliaire

Les résultats comparés de l'excrétion urinaire avec ceux de l'Hexabrix sont donnés dans le tableau II suivant

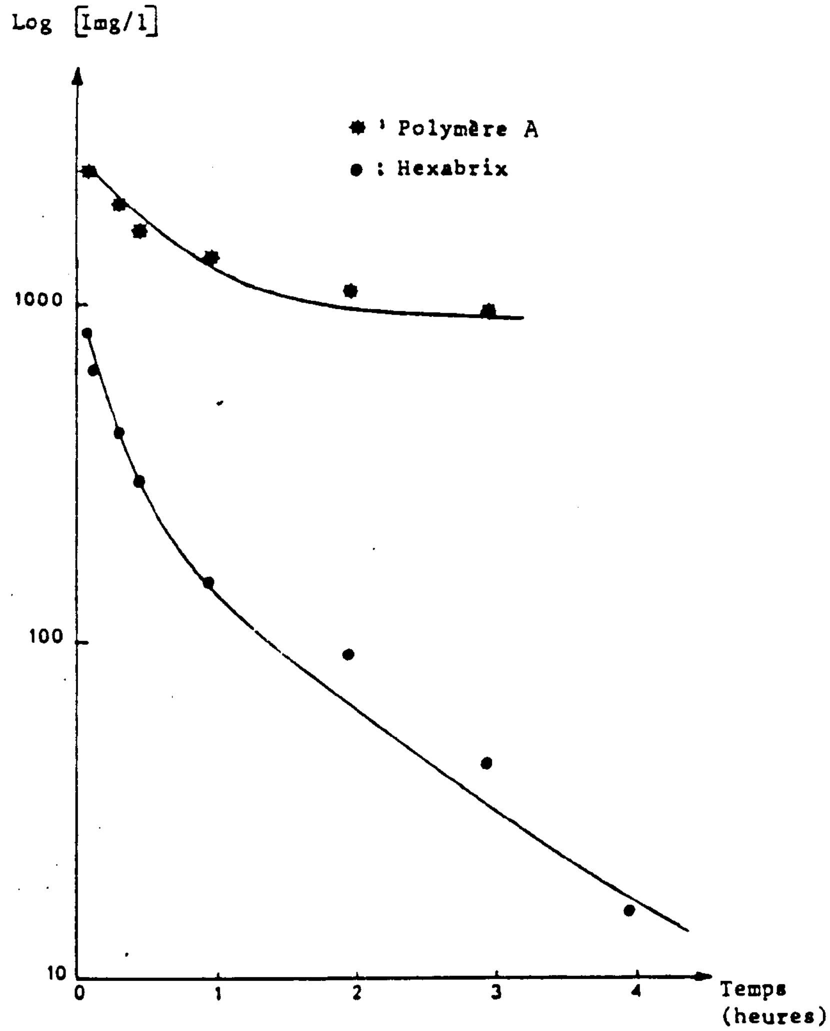


Graphique 1

Pourcentages d'excrétions urinaire en fonction du temps chez le lapin.

Tableau II

| | Temps (min) | Hexabrix % dose excrétée | Polymère A % dose excrétée |
|---|---|---|---|
| Excrétion urinaire | 0 - 30 | 30,63 ± 6,99 | 9,91 ± 6,43 |
| | 30 - 120 | 45,76 ± 8,33 | 21,75 ± 7,40 |
| | 120 - 240 | 49,39 ± 12,25 | 30,24 ± 0,14 |

Le polymère A est excrété par voie urinaire. En revanche l'excrétion biliaire est pratiquement nulle.

3) Osmolarité

On sait que l'osmolarité est un paramètre critique du point de vue des effets secondaires.
On donnera ci-après des résultats relatifs des polymères ainsi préparés.

| Exemple | Osmolarité mOsm/kg (20°C) |
|---|---|
| 5 | 489 |
| 6 | 559 |
| 10 | 43 |
| | (+ NaCl 497) |
| 19 | 183 |
| 20 | 60 |
| 21 | 106 (37°C) |

Ces résultats mettent en évidence des valeurs d'osmolarité acceptables et même plus faible que pour le plasma dans le cas de polymères à pont ester ou à pont derivant de l'épichlorhydrine.

La présente invention a également pour objet des produits de contraste qui comprennent au moins un polymère iodé tel que défini précédemment.

Ces produits de contraste sont utilisables chez l'homme et les animaux à des fins radiologiques.

La forme pharmaceutique préférée des produits de contraste selon l'invention est constituée par des solutions aqueuses des polymères.

On donnera ci-après un exemple de produit de contraste selon la présente invention

| Produit de contraste | |
|---|---|
| Polymère de l'exemple 10 | 20 g |
| Eau pour préparation injectable | |
| Q.S.P. | 100 ml |

D'une manière générale les produits de contraste selon l'invention peuvent être administré par toutes les voies classiquement utilisées pour les produits de contraste et en particulier par voie parentérale (voie intraveineuse, intra-artérielle, intra ou péri-lymphatique, voie sous-arachnoïdienne (myélographie)) et par voie orale.

Les produits de contraste selon la présente invention peuvent être utilisés en particulier en angiographie. On peut généralement les injecter chez l'homme à des doses d'iodes de 30 à 500 mgI/kg.

## Revendications

1. Polymères iodés utilisables en radiographie comme produits de contraste et caractérisés en ce qu'ils comprenent un squelette constitué par un dextrane sur lequel sont greffés des groupes de formule

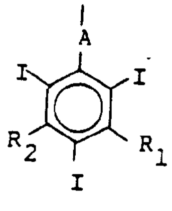

I

dans laquelle
A est un groupe formant un pont entre la chaine du dextrane et le noyau benzénique,
$R_1$ est un groupe choisi parmi le groupe COOH, le groupe COOH salifié par une base pharmaceutiquement acceptable et les groupes de formule

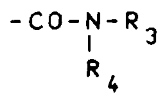

et

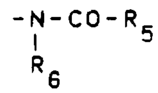

,

et
$R_2$ est un groupe choisi parmi les groupes de formule

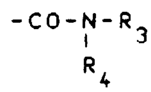

et

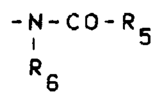

formules dans lesquelles
$R_3$ et $R_5$ sont choisis parmi les groupes alkyle en $C_1$ à $C_6$, hydroxyalkyle en $C_1$ à $C_6$, polyhydroxyalkyle en $C_1$ à $C_6$, alkoxy ($C_1$-$C_6$) alkyle ($C_1$-$C_6$) et alkoxy ($C_1$-$C_6$) hydroxyalkyle ($C_1$-$C_6$),
$R_4$ et $R_6$ sont choisis parmi un atome d'hydrogène et les groupes alkyle en $C_1$ à $C_6$, hydroxyalkyle en $C_1$ à $C_6$, polyhydroxyalkyle en $C_1$ à $C_6$, alkoxy ($C_1$-$C_6$) alkyle ($C_1$-$C_6$) et alkoxy ($C_1$-$C_6$) hydroxyalkyle ($C_1$-$C_6$),
$R_3$ pouvant être en outre un groupe de formule

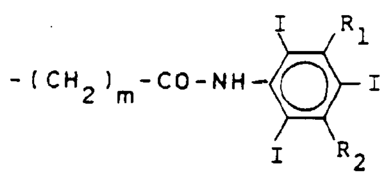

m étant un entier de 1 à 6 et
$R_1$ et $R_2$ ayant la signification donnée précédemment.

2. Polymère selon la revendication 1, caractérisé en ce qu'il comprend des groupes de formule

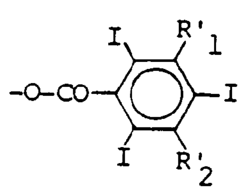

dans laquelle
$R'_1$ et $R'_2$ sont des groupes choisis parmi un groupe de formule

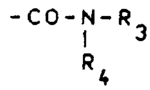

et un groupe de formule

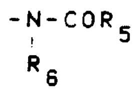

$R_3$, $R_4$, $R_5$ et $R_6$ ayant la signification donnée à la revendication 1.

3. Polymère selon la revendication 1, caractérisé en ce qu'il comprend des groupes de formule

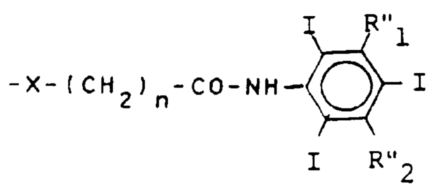

V

dans laquelle n est un entier de 1 à 5

$R''_1$ est un groupe -COOH, un groupe COOH salifié par une base pharmaceutiquement acceptable ou un groupe

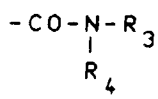

$R''_2$ est un groupe

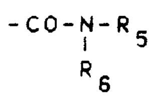

ou un groupe

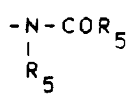

$R_3$, $R_4$, $R_5$ et $R_6$ ayant la signification donnée, X est un groupe choisi parmi
>N- , -NH- , -O-$CH_2$-CO-NH- ,

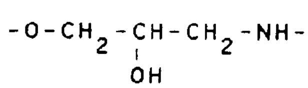

et
-OCO$CH_2CH_2$-CO-NH-

4. Polymère selon la revendication 1, caractérisé en ce qu'il comprend des groupes de formule

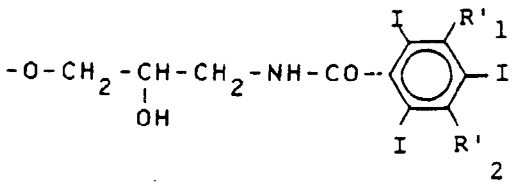

VI

dans laquelle
R′1, et R′2 sont des groupes choisis parmi un groupe de formule

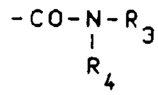

et un groupe de formule

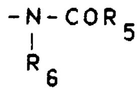

$R_3$, $R_4$, $R_5$ et $R_6$ ayant la signification donnée dans la revendication 1.

5. Procédé de préparation d'un polymère selon la revendication 2, caractérisé en ce que l'on fait réagir sur un dextrane un chlorure d'acide de formule

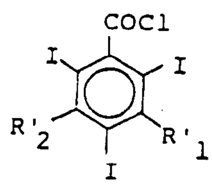

IIa

$R'_1$ et $R'_2$ ayant la signification donnée à la revendication 2.

6. Procédé de préparation d'un polymère selon la revendication 3 dans lequel X est un groupe >N-, caractérisé en ce que l'on fait réagir le périodate de sodium sur un dextrane puis on'fait réagir sur le dialdéhydodextrane obtenu une amine de formule

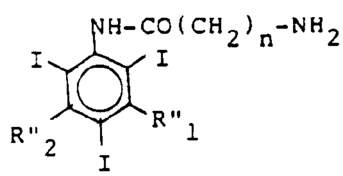

$IV_a$

dans laquelle n,$R''_1$ et $R''_2$ ont la signification donnée à la revendication 3.

7. Procédé de préparation d'un polymère selon la revendication 3, dans lequel X est un groupe -NH-, caractérisé en ce que l'on fait réagir le chlorure de mésyle sur un dextrane, puis on fait réagir sur le polymère obtenu une amine de formule

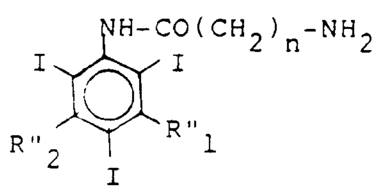

$IV_a$

dans laquelle n, $R''_1$ et $R''_2$ ont la signification donnée à la revendication 3.

8. Procédé de préparation d'un polymère selon la revendication 3, dans lequel X est un groupe $-OCH_2-$

CO-NH-, caractérisé en ce que l'on fait réagir l'acide monochloroacétique en milieu alcalin sur un dextrane puis l'on fait réagir sur le carboxyméthyldextrane obtenu une amine de formule

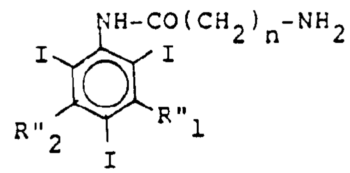

$IV_a$

dans laquelle n, $R''_1$ et $R''_2$ ont la signification donnée à la revendication 3.

9. Procédé de préparation d'un polymère selon la revendication 3, dans lequel X est un groupe

$$-O-CH_2-CH(OH)-CH_2-NH-,$$

caractérisé en ce que l'on fait réagir l'épichlorhydrine en milieu alcalin ou l'épichlorhydrine en présence de Zn $(BF_4)_2$ sur un dextrane, puis l'on fait réagir sur le polymère obtenu une amine de formule

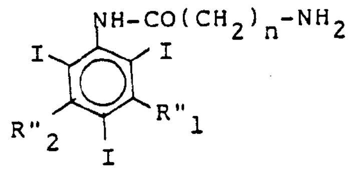

$IV_a$

dans laquelle n, $R''_1$ et $R''_2$ ont la signification donnée à la revendication 3.

10. Procédé de préparation d'un polymère selon la revendication 3, dans lequel X est un groupe -O-CO-$CH_2$-$CH_2$-CO-NH-, caractérisé en ce que l'on fait réagir l'anhydride succinique sur un dextrane, puis l'on fait réagir sur le polymère obtenu une amine de formule

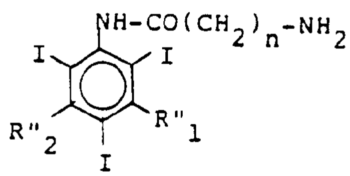

$IV_a$

dans laquelle n, $R''_1$ et $R''_2$ ont la signification donnée à la revendication 3.

11. Procédé de préparation d'un polymère selon la revendication 4, caractérisé en ce que l'on fait réagir l'épichlorhydrine en présence de Zn $(BF_4)_2$ sur le dextrane puis l'on fait réagir sur le polymère l'ammoniaque puis un chlorure d'acide de formule

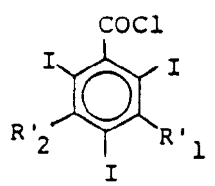

IIa

$R'_1$ et $R'_2$ ayant la signification donnée à la revendication 4.

12. Produit de contraste pour la radiographie, caractérisé en ce qu'il contient un polymère selon l'une quelconque des revendications 1 à 4.

13. Produit de contraste selon la revendication 12, caractérisé en ce qu'il est constitué par une solution aqueuse du polymère.

## Claims

1. Iodinated polymers, which can be used in radiography as contrast media, characterised in that they comprise a skeleton consisting of a dextran onto which are grafted groups of formula

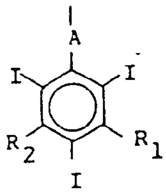

I

in which
A is a group forming a bridge between the dextran chain and the benzene ring,
$R_1$ is a group selected from the COOH group, the COOH group salified with a pharmaceutically acceptable base and the groups of formula

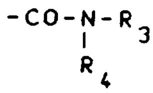

and

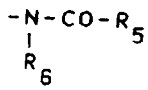

,

and
$R_2$ is a group chosen from the groups of formula

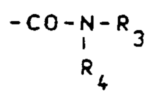

and

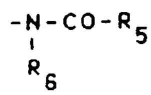

in which
$R_3$ and $R_5$ are selected from $C_1$ to $C_6$ alkyl, $C_1$ to $C_6$ hydroxyalkyl, $C_1$ to $C_6$ polyhydroxyalkyl, ($C_1$ - $C_6$) alkoxy ($C_1$ - $C_6$) alkyl and ($C_1$ - $C_6$) alkoxy ($C_1$ to $C_6$) hydroxyalkyl groups,
$R_4$ and $R_6$ are selected from a hydrogen atom and $C_1$ to $C_6$ alkyl, $C_1$ to $C_6$ hydroxyalkyl, $C_1$ to $C_6$ polyhydroxyalkyl, ($C_1$ - $C_6$) alkoxy ($C_1$-$C_6$) alkyl and ($C_1$-$C_6$) alkoxy ($C_1$ to $C_6$) hydroxyalkyl groups, it being possible for $R_3$ to be, in addition, a group of formula

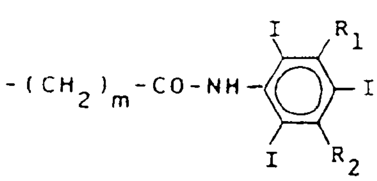

m being an integer from 1 to 6, and
$R_1$ and $R_2$ having the meaning given above.

2. Polymer according to claim 1, characterised in that it comprises groups of formula

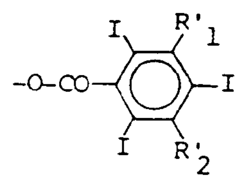

in which
$R'_1$ and $R'_2$ are groups chosen from a group of formula

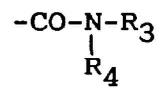

and a group of formula

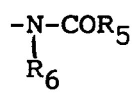

$R_3$, $R_4$, $R_5$ and $R_6$ having the meaning given in claim 1.

3. Polymer according to claim 1, characterised in that it comprises groups of formula

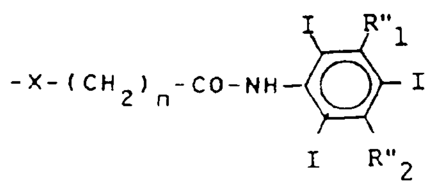

V

in which n is an integer from 1 to 5
$R''_1$ is a -COOH group, a -COOH group salified with a pharmaceutically acceptable base or a

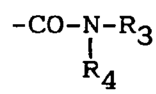

group
$R''_2$ is a

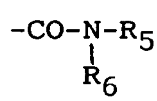

group or a

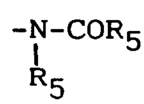

group
$R_3$, $R_4$, $R_5$ and $R_6$ having the meaning given, and X is a group chosen from
>N-, -NH-, -O-$CH_2$-CO-NH-,

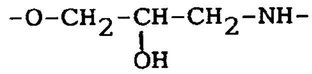

and
$-OCOCH_2-CH_2-CO-NH-$

4. Polymer according to claim 1, characterised in that it comprises groups of formula

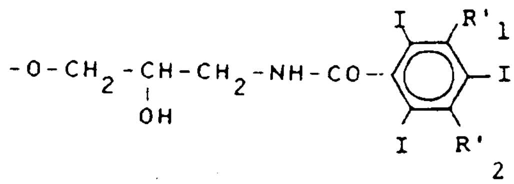

VI

in which
$R'_1$ and $R'_2$ are groups chosen from a group of formula

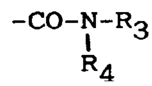

and a group of formula

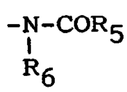

$R_3$, $R_4$, $R_5$, and $R_6$ having the meaning given in claim 1.

5. Process for preparing a polymer according to claim 2, characterised in that an acid chloride of formula

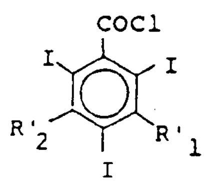

IIa

$R'_1$ and $R'_2$ having the meaning given in claim 2, is reacted with a dextran.

6. Process for preparing a polymer according to claim 3 in which X is an ›N-group, characterised in that sodium periodate is reacted with a dextran and the dialdehydodextran obtained is then reacted with an amine of formula

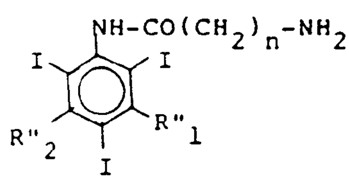

$IV_a$

in which n, $R''_1$ and $R''_2$ have the meaning given in claim 3.

7. Process for preparing a polymer according to claim 3 in which X is an -NH- group, characterised in that mesyl chloride is reacted with dextran and the polymer obtained is then reacted with an amine of formula

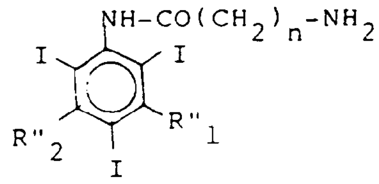

IV$_a$

in which n, $R''_1$ and $R''_2$ have the meaning given in claim 3.

8. Process for preparing a polymer according to claim 3 in which X is an $-OCH_2-CO-NH-$ group, characterised in that monochloroacetic acid in an alkaline medium is reacted with a dextran and the carboxymethyldextran obtained is then reacted with an amine of formula

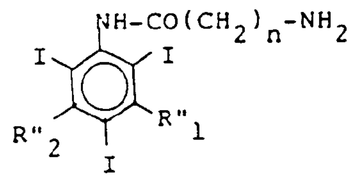

IV$_a$

in which n, $R''_1$ and $R''_2$ have the meaning given in claim 3.

9. Process for preparing a polymer according to claim 3 in which X is an

$$-O-CH_2-\underset{OH}{\underset{|}{CH}}-CH_2-NH-$$

group characterised in that epichlorohydrin in an alkaline medium or epichlorohydrin in the presence of $Zn(BF_4)_2$ is reacted with a dextran and the polymer obtained is then reacted with an amine of formula

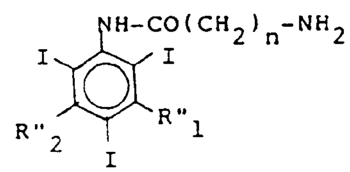

IV$_a$

in which n, $R''_1$ and $R''_2$ have the meaning given in claim 3.

10. Process for preparing a polymer according to claim 3, in which X is an $-O-CO-CH_2-CH_2-CO-NH-$ group, charactereised in that succinic anhydride is reacted with a dextran and the polymer obtained is then reacted with an amine of formula

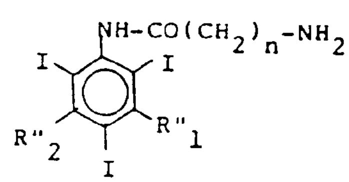

IV$_a$

in which n, $R''_1$ and $R''_2$ have the meaning given in claim 3.

11. Process for preparing a polymer according to claim 4, charactersed in that epichlorohydrin in the presence of $Zn(BF_4)_2$ is reacted with dextran, and ammonia solution and then acid chloride of formula

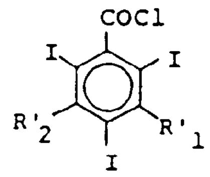

IIa

$R'_1$ and $R'_2$ having the meaning given in claim 4, are then reacted with the polymer.

12. Contrast medium for radiography, characterised in that it contains a polymer according to any one of claims 1 to 4.

13. Contrast medium according to claim 12, characterised in that it comprises an aqueous solution of the polymer.

## Patentansprüche

1. Jodierte Polymere, die in der Röntgenographie als Kontrastmittel verwendbar sind, dadurch gekennzeichnet, daβ sie ein Gerüst besitzen, das aus einem Dextran besteht, auf das Gruppen der Formel

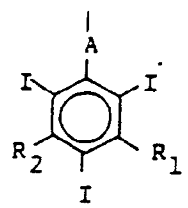

I

aufgepfropft sind, in der

A eine Gruppe ist, die eine Brücke zwischen der Kette des Dextrans und dem Benzolring bildet,

$R_1$ eine Gruppe ist, die ausgewählt ist aus der COOH-Gruppe, der durch eine pharmazeutisch annehmbare Base in ein Salz überführten COOH-Gruppe und den Gruppen der Formel

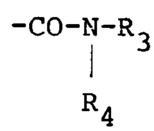

und

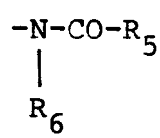

und

$R_2$ eine Gruppe ist, die ausgewählt ist aus den Gruppen der Formel

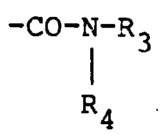

und

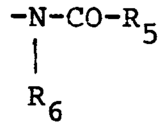

wobei in diesen Formeln
$R_3$ und $R_5$ aus den $C_1$-$C_6$-Alkyl-, $C_1$-$C_6$-Hydroxyalkyl-, $C_1$-$C_6$-Polyhydroxyalkyl-, ($C_1$-$C_6$)Alkoxy-($C_1$-$C_6$)Alkyl- und($C_1$-$C_6$)Alkoxy-($C_1$-$C_6$)-Hydroxyalkyl-Gruppen,
$R_4$ und $R_6$ ausgewählt sind aus einem Wasserstoffatom und den $C_1$-$C_6$-Alkyl-, $C_1$-$C_6$-Hydroxyalkyl-, $C_1$-$C_6$-Polyhydroxyalkyl-, ($C_1$-$C_6$)Alkoxy-($C_1$-$C_6$)-Alkyl- und($C_1$-$C_6$) Alkoxy-($C_1$-$C_6$)-Hydroxyalkyl-Gruppen,
$R_3$ ferner eine Gruppe der Formel

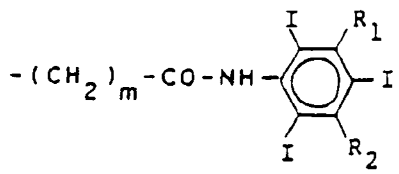

sein kann,
m eine ganze Zahl von 1 bis 6 ist und
$R_1$ und $R_2$ die obengenannten Bedeutungen haben.

2. Polymer nach Anspruch 1, dadurch gekennzeichnet, daβ es Gruppen der Formel

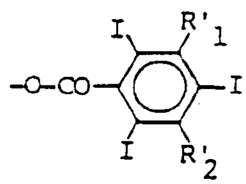

umfaβt, in der
$R'_1$ und $R'_2$ Gruppen sind, die ausgewählt sind aus einer Gruppe der Formel

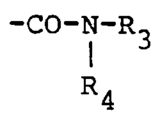

und einer Gruppe der Formel

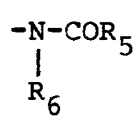 ,

$R_3$, $R_4$, $R_5$ und $R_6$ die in Anspruch 1 genannten Bedeutungen haben.

3. Polymer nach Anspruch 1, dadurch gekennzeichnet, daβ es Gruppen der Formel

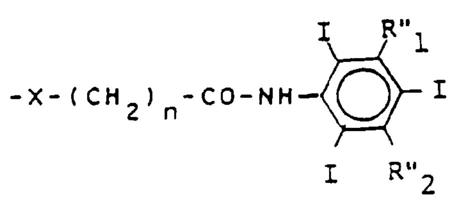

V

umfaßt,
in der n eine ganze Zahl von 1 bis 5,
R″$_1$ eine -COOH-Gruppe, eine durch eine pharmazeutisch annehmbare Base in ein Salz überführte COOH-Gruppe oder eine

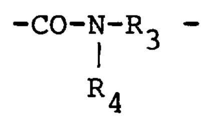

Gruppe ist,
R″ $_2$ eine

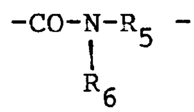

Gruppe oder eine

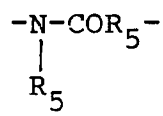

Gruppe ist,
$R_3$, $R_4$, $R_5$ und $R_6$ die genannten Bedeutungen haben und X eine Gruppe ist, die ausgewählt ist aus >N-, -NH-, -O-$CH_2$-CO-NH-,

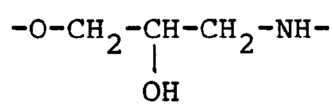

und -OCO$CH_2CH_2$-CO-NH- .

4. Polymer nach Anspruch 1, dadurch gekennzeichnet, daß es Gruppen der Formel

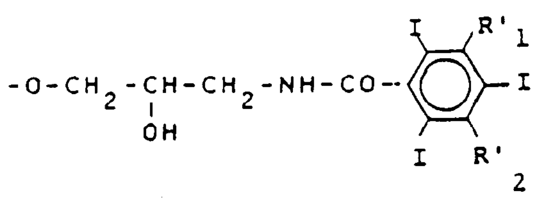 VI

umfaßt, in der
R′$_1$ und R′$_2$ Gruppen sind, die ausgewählt sind aus einer Gruppe der Formel

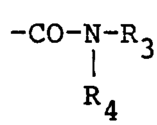

und einer Gruppe der Formel

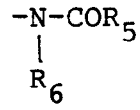

$R_3$, $R_4$, $R_5$ und $R_6$ die in Anspruch 1 genannten Bedeutungen haben.

5. Verfahren zur Herstellung eines Polymers nach Anspruch 2, dadurch gekennzeichnet, daβ man mit einem Dextran ein Säurechlorid der Formel

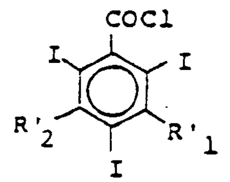

IIa

reagieren läβt, worin $R'_1$ und $R'_2$ die in Anspruch 2 genannten Bedeutungen haben.

6. Verfahren zur Herstellung eines Polymers nach Anspruch 3, bei dem X eine >N--Gruppe ist, dadurch gekennzeichnet, daβ man das Natriumperjodat mit einem Dextran reagieren läβt und daβ man dann mit dem erhaltenen Dialdehydodextran ein Amin der Formel

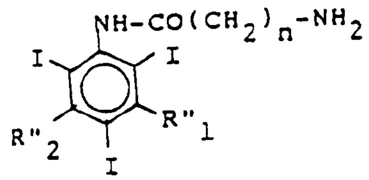

$IV_a$

reagieren läβt, in der n, $R''_1$ und $R''_2$ die in Anspruch 3 genannten Bedeutungen haben.

7. Verfahren zur Herstellung eines Polymers nach Anspruch 3, bei dem X eine -NH--Gruppe ist, dadurch gekennzeichnet, daβ man Mesylchlorid mit einem Dextran reagieren läβt und dann mit dem erhaltenen Polymer ein Amin der Formel

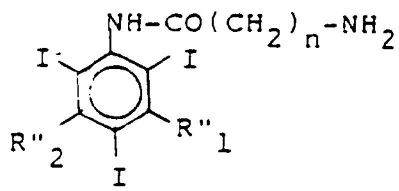

$IV_a$

reagieren läβt, in der n, $R''_1$ und $R''_2$ die in Anspruch 3 genannten Bedeutungen haben.

8. Verfahren zur Herstellung eines Polymers nach Anspruch 3, bei dem X eine $-OCH_2-CO-NH-$-Gruppe ist, dadurch gekennzeichnet, daβ man Monochloressigsäure im alkalischen Milieu mit einem Dextran reagieren läβt und dann mit dem erhaltenen Carboxymethyldextran ein Amin der Formel

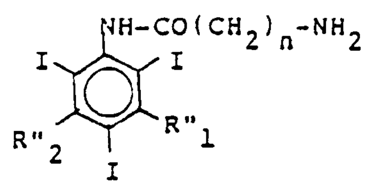

$IV_a$

reagieren läβt, in der n, $R''_1$ und $R''_2$ die in Anspruch 3 genannten Bedeutungen haben.

9. Verfahren zur Herstellung eines Polymers nach Anspruch 3, bei dem X eine

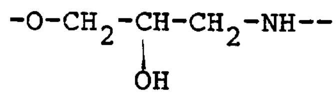

Gruppe ist, dadurch gekennzeichnet, daß man Epichlorhydrin in alkalischem Milieu oder Epichlorhydrin in Anwesenheit von Zn $(BF_4)_2$ mit einem Dextran reagieren läßt und dann mit dem erhaltenen Polymer ein Amin der Formel

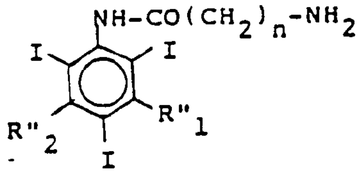

IV$_a$

reagieren läßt, in der n, $R''_1$ und $R''_2$ die in Anspruch 3 genannten Bedeutungen haben.

10. Verfahren zur Herstellung eines Polymers nach Anspruch 3, bei dem X eine -O-CO-$CH_2$-$CH_2$-CO-NH- -Gruppe ist, dadurch gekennzeichnet, daß man Bernsteinsäureanhydrid mit einem Dextran reagieren läßt und dann mit dem erhaltenen Polymer ein Amin der Formel

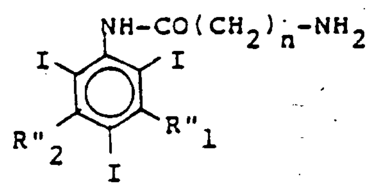

IV$_a$

reagieren läßt, in der n, $R''_1$ und $R''_2$ die in Anspruch 3 genannten Bedeutungen haben.

11. Verfahren zur Herstellung eines Polymers nach Anspruch 4, dadurch gekennzeichnet, daß man Epichlorhydrin in Anwesenheit von Zn $(BF_4)_2$ mit Dextran reagieren läßt und dann mit dem Polymer wässrige Lösung von Ammoniak und dann ein Säurechlorid der Formel

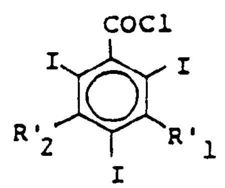

IIa

reagieren läßt, worin $R'_1$ und $R'_2$ die in Anspruch 4 genannten Bedeutungen haben.

12. Kontrastmittel zur Röntgenographie, dadurch gekennzeichnet, daß es ein Polymer nach einem der Ansprüche 1 bis 4 enthält.

13. Kontrastmittel nach Anspruch 12, dadurch gekennzeichnet, daß es aus einer wässrigen Lösung des Polyers besteht.